# EUROPEAN PATENT SPECIFICATION

(11) **EP 4 031 217 B1**
(45) Date of publication and mention of the grant of the patent: **26.03.2025**
(21) Application number: 19956917.9
(22) Date of filing: 15.12.2019
(51) Int. Cl.: A61M 15/00, B05B 17/06, A61M 15/06, A24F 40/05, A24F 40/10, A61M 11/00

(54) **ULTRASONIC MIST INHALER**
ULTRASCHALLNEBELINHALATOR
INHALATEUR DE BRUME À ULTRASONS

(43) Date of publication of application: 27.07.2022
(73) Proprietor: Shaheen Innovations Holding Limited, Abu Dhabi (AE)
(72) Inventor: ALSHAIBA SALEH GHANNAM ALMAZROUEI, Mohammed, Abu Dhabi (AE)
(74) Representative: Forresters IP LLP
(86) International application number: PCT/IB2019/060809
(87) International publication number: WO 2021/123868

(56) References cited:
- EP-A1- 3 545 778
- EP-A1- 3 545 778
- WO-A1-2019/052506
- CN-U- 206 303 211
- CN-U- 206 303 211

## Description

### FIELD OF THE DISCLOSED TECHNOLOGY

The invention relates to an ultrasonic mist inhaler for atomizing a liquid by ultrasonic vibrations.

### BACKGROUND

Electronic vaporizing inhalers are becoming popular among smokers who also want to avoid the tar and other harsh chemicals associated with traditional cigarettes and who wish to satisfy the craving for nicotine. Electronic vaporizing inhalers may contain liquid nicotine, which is typically a mixture of nicotine oil, a solvent, water, and often flavoring. When the user draws, or inhales, on the electronic vaporizing inhaler, the liquid nicotine is drawn into a vaporizer where it is heated into a vapor. As the user draws on the electronic vaporizing inhaler, the vapor containing the nicotine is inhaled. Such electronic vaporizing inhalers may have medical purpose.

Electronic vaporizing inhalers and other vapor inhalers typically have similar designs. Most electronic vaporizing inhalers feature a liquid nicotine reservoir with an interior membrane, such as a capillary element, typically cotton, that holds the liquid nicotine so as to prevent leaking from the reservoir. Nevertheless, these cigarettes are still prone to leaking because there is no obstacle to prevent the liquid from flowing out of the membrane and into the mouthpiece. A leaking electronic vaporizing inhaler is problematic for several reasons. As a first disadvantage, the liquid can leak into the electronic components, which can cause serious damage to the device. As a second disadvantage, the liquid can leak into the electronic vaporizing inhaler mouthpiece, and the user may inhale the unvaporized liquid.

Electronic vaporizing inhalers are also known for providing inconsistent doses between draws. The aforementioned leaking is one cause of inconsistent doses because the membrane may be oversaturated or undersaturated near the vaporizer. If the membrane is oversaturated, then the user may experience a stronger than desired dose of vapor, and if the membrane is undersaturated, then the user may experience a weaker than desired dose of vapor. Additionally, small changes in the strength of the user's draw may provide stronger or weaker doses. Inconsistent dosing, along with leaking, can lead to faster consumption of the vaping liquid.

Additionally, conventional electronic vaporizing inhalers tend to rely on inducing high temperatures of a metal heating component configured to heat a liquid in the e-cigarette, thus vaporizing the liquid that can be breathed in. Problems with conventional electronic vaporizing inhalers may include the possibility of burning metal and subsequent breathing in of the metal along with the burnt liquid. In addition, some may not prefer the burnt smell caused by the heated liquid.

Electronic vaporizing inhalers are generally designed so that the liquid nicotine reservoir is arranged away from the metal heating component to prevent heating the unused liquid in the reservoir. This arrangement makes the inhaler device more complex to produce.

Thus, a need exists in the art for an electronic vaporizing inhaler that is better able to withstand these disadvantages.

EP 3545778 discloses an atomizer and electronic cigarette inclined ultrasonic atomizing sheet structure.

WO2019/052506 discloses an ultrasonic electronic cigarette atomizer with an air intake tube, an atomization area of an ultrasonic atomization sheet, an air discharge channel and a suction nozzle in communication in sequence.

### BRIEF SUMMARY

According to one aspect of the invention, there is provided an ultrasonic mist inhaler as defined in claim 1 hereafter.

The airflow member permits ambient air to have fluid contact with means of ultrasonic vibration when the user draws from the mouthpiece. Using a sonication chamber for ultrasonic vibrations in an ultrasonic mist inhaler allows the combination of the liquid chamber and the sonication chamber into the liquid reservoir structure.

The expression "means of ultrasonic vibrations" is similar to the expression "ultrasonic oscillation component" used in the patent application PCT/IB2019/055192.

It is noted that the expression "mist" used in the invention means the liquid is not heated as usually in traditional inhalers known from the prior art. In fact, traditional inhalers use heating elements to heat the liquid above its boiling temperature to produce a vapor, which is different from a mist.

In fact, when sonicating liquids at high intensities, the sound waves that propagate into the liquid media result in alternating high-pressure (compression) and low-pressure (rarefaction) cycles, at different rates depending on the frequency. During the low-pressure cycle, high-intensity ultrasonic waves create small vacuum bubbles or voids in the liquid. When the bubbles attain a volume at which they can no longer absorb energy, they collapse violently during a high-pressure cycle. This phenomenon is termed cavitation. During the implosion very high pressures are reached locally. At cavitation, broken capillary waves are generated, and tiny droplets break the surface tension of the liquid and are quickly released into the air, taking mist form.

In the ultrasonic mist inhaler, the airflow member comprises an airflow bridge and an airflow duct, the airflow bridge having at least an airway opening forming a portion of the inhalation channel and the airflow duct extending in the sonication chamber from the airflow bridge for providing the air flow from the surroundings to the sonication chamber.

The airflow member allows the air-mist mix to exit upwards through the airway opening to the mouthpiece. Further, the airflow bridge prevents any large liquid drops from being expelled through the mouthpiece.

Preferably, the airflow bridge comprises two semicircular airway openings.

According to the invention, airflow bridge is engaged with a frustoconical element.

In the ultrasonic mist inhaler, a capillary element may be arranged between the liquid chamber and the sonication chamber.

In the ultrasonic mist inhaler, the airflow duct may face the capillary element.

In the ultrasonic mist inhaler, the airflow bridge comprises at least a peripheral opening providing air flow to the airflow duct.

In the ultrasonic mist inhaler, the frustoconical element comprises at least a peripheral opening aligned with the peripheral opening of the airflow bridge.

In the ultrasonic mist inhaler, wherein at least part of the liquid reservoir structure and the mouthpiece form the sonication chamber.

In the ultrasonic mist inhaler, it comprises a mouthpiece for inhaling the mist.

In the ultrasonic mist inhaler, an airflow chamber is arranged between the mouthpiece and the frustoconical element.

In the ultrasonic mist inhaler, the mouthpiece may comprise at least a peripheral opening aligned with the peripheral opening of the airflow bridge and the peripheral opening of the frustoconical element.

The end of the airflow duct may extend in the sonication chamber and is close to or at the capillary element.

In the ultrasonic mist inhaler, the airflow bridge and the airflow duct may be made in one piece.

In the ultrasonic mist inhaler, the liquid reservoir structure comprises an inhalation channel providing an air passage from the sonication chamber toward the surroundings.

In the ultrasonic mist inhaler, the inhalation channel may comprise a frustoconical element arranged between the mouthpiece and the liquid reservoir structure.

In the ultrasonic mist inhaler, the inhalation channel may comprise an inner container delimiting the sonication chamber and the liquid chamber.

In the ultrasonic mist inhaler, the inner container may form the inner wall of the liquid chamber.

In the ultrasonic mist inhaler, the frustoconical element may be in contact with the inner container.

In the ultrasonic mist inhaler, the mouthpiece may comprise an inner duct receiving the frustoconical element.

In the ultrasonic mist inhaler, the liquid reservoir structure may comprise an outer container delimiting the outer wall of the liquid chamber.

In the ultrasonic mist inhaler, the inner container may comprise a first section and a second section between which the capillary element is arranged.

The capillary element may be a gauze. The capillary element may be formed of bamboo fibers, preferably in 100% bamboo fibers, or cotton, silica, tobacco cotton, or any combination of such material.

In the ultrasonic mist inhaler, the capillary element may have a flat shape extending from the sonication chamber to the liquid chamber.

In the ultrasonic mist inhaler, the capillary element may comprise a central portion and a peripheral portion.

In the ultrasonic mist inhaler, the peripheral portion may have an L-shape cross section extending down to the liquid chamber.

In the ultrasonic mist inhaler, the central portion may have a U-shape cross section extending down to the sonication chamber.

In the ultrasonic mist inhaler, the sonication chamber comprises means of ultrasonic vibrations.

In the ultrasonic mist inhaler, the means of ultrasonic vibrations are supported by an elastic member.

The elastic member is formed from an annular plate-shaped rubber.

The elastic member has an inner hole wherein a groove is designed for maintaining the means of ultrasonic vibrations.

In this case, since the elastic member is in line contact with the means of ultrasonic vibrations, suppression of vibrations of the liquid reservoir structure can more effectively be prevented. Thus, fine particles of the liquid atomized by the atomizing member can be sprayed farther.

In the ultrasonic mist inhaler, a high-frequency voltage may be applied to the means of ultrasonic vibrations to ultrasonically vibrate the means of ultrasonic vibrations, whereby a liquid supplied to a portion of the means of ultrasonic vibrations can be atomized and sprayed.

According to the ultrasonic mist inhaler, fine particles of the liquid atomized by the means of ultrasonic vibrations having a relatively small size can be sprayed farther.

Such means of ultrasonic vibrations may be a transducer, preferably designed in a circular plate-shape. The material of the piezoelectric transducer is preferably in ceramic.

In the ultrasonic mist inhaler, the U-shape cross section may have an inner portion and an outer portion, the inner portion of the U-shape cross section being in surface contact with the means of ultrasonic vibrations and the outer portion of the U-shape cross section being not in surface contact with the means of ultrasonic vibrations.

In the ultrasonic mist inhaler, the outer portion of the U-shape cross section may have airflow apertures through which may pass the mist produced between the outer portion of the U-shape cross section and the means of ultrasonic vibrations.

In the ultrasonic mist inhaler, the inner container may be configured to receive a mechanical spring.

Preferably, the mechanical spring pushes the central portion of the capillary element onto the means of ultrasonic vibrations to ensure the surface contact.

In the ultrasonic mist inhaler, the sonication chamber may have a bottom plate closing the sonication chamber.

In the ultrasonic mist inhaler, the bottom plate may be configured to support the elastic member.

In the ultrasonic mist inhaler, the bottom plate may comprise an upper surface having a recess wherein the elastic member is received.

In the ultrasonic mist inhaler, the liquid chamber may comprise a top surface representing the maximum level of the liquid chamber and the bottom surface representing the minimum level of the liquid, and wherein the bottom surface being the upper surface of the bottom plate.

In the ultrasonic mist inhaler, the liquid reservoir structure may comprise a cap closing the liquid chamber.

In the ultrasonic mist inhaler, the cap may comprise a lower surface, the top surface of the liquid chamber being the lower surface of the cap.

In the ultrasonic mist inhaler, the bottom plate may be arranged to receive connectors for powering the means of ultrasonic vibrations.

In the ultrasonic mist inhaler, a first connector and a second connector, the first connector is a spring-loaded contact probe and the second connector is a side pin crossing the elastic member. A spring-loaded contact probe provides a permanent contact with the means of ultrasonic vibrations.

Preferably, a metal plate ensures electrical contact between the second connector and the means of ultrasonic vibrations.

In the ultrasonic mist inhaler, the liquid reservoir structure may have a top end comprising an airway fluidically coupled to the internal bore and configured to allow liquid mist to flow out of the airway.

In the ultrasonic mist inhaler, the liquid reservoir structure may be arranged between a mouthpiece and a casing housing electrical components for powering and operating the inhaler.

The bottom plate is sealed, thus preventing leakage of liquid from the sonication chamber to the casing.

In the ultrasonic mist inhaler, the top end of the liquid reservoir structure may be coupled with the mouthpiece via the frustoconical element, the mouthpiece being configured to allow suction of liquid mist flowing out of the airway to the surroundings.

Advantageously, the liquid reservoir structure is detachable from the mouthpiece and the casing.

The liquid reservoir structure and the mouthpiece or the casing may include complimentary arrangements for engaging with one another; further such complimentary arrangements may include; a bayonet type arrangement; a threaded engaged type arrangement; a magnetic arrangement; and, a friction fit arrangement; wherein the liquid reservoir structure includes a portion of the arrangement and the mouthpiece or the casing includes the complimentary portion of the arrangement.

Such design permits the liquid reservoir structure to be disposable. The liquid reservoir structure may be removed and reinstalled when required; a feature that gives the user the freedom to interchange flavors without the limitation of either completely using or having to discard unused liquid.

Further, such design eliminates the risk of fatigue of the means of ultrasonic vibrations in the sonication chamber and has a disposable means of ultrasonic vibrations which prevents the risk of fatigue and a disposable capillary element which prevents the risk of mixing flavor.

In the ultrasonic mist inhaler, an integrated circuit may be coupled to the bottom end of the liquid reservoir structure and communicatively coupled to the means of ultrasonic vibrations, the integrated circuit configured to cause the means of ultrasonic vibrations to vibrate.

Preferably, the means of ultrasonic vibrations is in electrical communication with an integrated circuit, preferably via the connectors.

Advantageously, the integrated circuit is designed to convert a direct current into an alternate current at high frequency.

The means of ultrasonic vibrations may be powered from electrical components of the casing through the connectors.

Preferably, the casing is configured to contain an electrical storage device and may encase at least a portion of the integrated circuit.

The casing may have all features described in the patent application PCT/IB2019/055192.

The ultrasonic mist inhaler according to the invention, wherein said liquid to be received in the liquid chamber comprises 57-70 % (w/w) vegetable glycerin and 30-43% (w/w) propylene glycol, said propylene glycol including nicotine and flavorings.

An ultrasonic mist inhaler or a personal ultrasonic atomizer device, comprising:
- a liquid reservoir structure comprising a liquid chamber or cartridge adapted to receive liquid to be atomized,
- a sonication chamber in fluid communication with the liquid chamber or cartridge,
wherein said liquid to be received in the liquid chamber comprises 57-70 % (w/w) vegetable glycerin and 30-43% (w/w) propylene glycol, said propylene glycol including nicotine and flavorings.

### BRIEF DESCRIPTION OF THE DRAWINGS

Some embodiments are illustrated by way of example and not limitation in the figures of the accompanying drawings.
FIG. 1 is an exploded view of components of the ultrasonic mist inhaler according to an embodiment of the invention.
FIG. 2 is an exploded view of components of the inhaler liquid reservoir structure according to an embodiment of the invention.
FIG. 3 is a cross section view of components of the inhaler liquid reservoir structure according to figure 1.
FIG. 4A is an isometric view of an airflow member of the inhaler liquid reservoir structure according to figures 2 and 3.
FIG. 4B is a cross section view of the airflow member shown in figure 4A.
FIG. 5A is an isometric view of another embodiment of the airflow member depicted in figures 4A and 4 B.
FIG. 5B is a cross section view of the airflow member shown in figure 5A.

### DETAILED DESCRIPTION

The foregoing summary, as well as the following detailed description of certain embodiments of the present invention, will be better understood when read in conjunction with the appended drawings.

As used herein, an element recited in the singular and preceded with the word "a" or "an" should be understood as not excluding plural of said elements, unless such exclusion is explicitly stated. Furthermore, the references to "one embodiment" of the present invention are not intended to be interpreted as excluding the existence of additional embodiments that also incorporate the recited features. Moreover, unless explicitly stated to the contrary, embodiments "comprising" or "having" an element or a plurality of elements having a particular property may include additional such elements not having that property.

The present invention is directed to an ultrasonic mist inhaler. The description of the invention and accompanying figures will be directed to the electronic vaporizing inhaler embodiment; however, other embodiments are envisioned, such as an inhaler for hookah, flavored liquids, medicine, and herbal supplements. Additionally, the device can be packaged to look like an object other than a cigarette. For instance, the device could resemble another smoking instrument, such as a pipe, water pipe, or slide, or the device could resemble another non-smoking related object.

Ultrasonic mist inhalers are either disposable or reusable. The term "reusable" as used herein implies that the energy storage device is rechargeable or replaceable or that the liquid is able to be replenished either through refilling or through replacement of the liquid reservoir structure. Alternatively, in some embodiments reusable electronic device is both rechargeable and the liquid can be replenished. A disposable embodiment will be described first, followed by a description of a reusable embodiment.

Conventional electronic vaporizing inhaler tend to rely on inducing high temperatures of a metal component configured to heat a liquid in the inhaler, thus vaporizing the liquid that can be breathed in. The liquid typically contains nicotine and flavorings blended into a solution of propylene glycol (PG) and vegetable glycerin (VG), which is vaporized via a heating component at high temperatures. Problems with conventional inhaler may include the possibility of burning metal and subsequent breathing in of the metal along with the burnt liquid. In addition, some may not prefer the burnt smell and taste caused by the heated liquid.

In contrast, aspects of the present disclosure include an ultrasonic mist inhaler that atomizes the liquid through ultrasonic vibrations, which produces micro water bubbles in the liquid. When the bubbles come into contact with ambient air, water droplets of about 0.25 to 0.5 microns spray into the air, thereby generating micro-droplets that can be absorbed through breathing, similar to breathing in a mist.

No heating elements are involved, thereby leading to no burnt elements and reducing second-hand smoke effects.

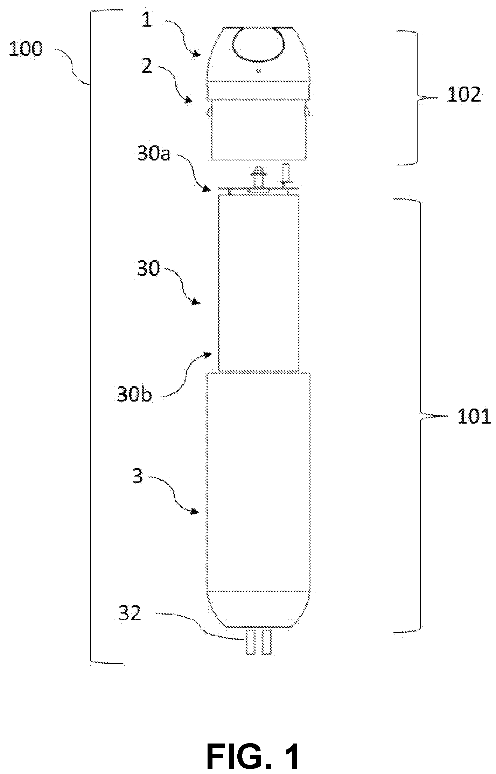

FIG. 1 depicts a disposable ultrasonic mist inhaler embodiment 100 of the invention. As can be seen in FIG. 1, the ultrasonic mist inhaler 100 has a cylindrical body with a relatively long length as compared to the diameter. In terms of shape and appearance, the ultrasonic mist inhaler 100 is designed to mimic the look of a typical cigarette. For instance, the inhaler can feature a first portion 101 that primarily simulates the tobacco rod portion of a cigarette and a second portion 102 that primarily simulates a filter. In the disposable embodiment of the invented device, the first portion and second portion are regions of a single, but-separable device. The designation of a first portion 101 and a second portion 102 is used to conveniently differentiate the components that are primarily contained in each portion.

As can be seen in FIG. 1, the ultrasonic mist inhaler comprises a mouthpiece 1, a liquid reservoir structure 2 and a casing 3. The first portion 101 comprises the casing 3 and the second portion 102 comprises the mouthpiece 1 and the reservoir structure 2.

The first portion 101 contains the power supply energy.

An electrical storage device 30 powers the ultrasonic mist inhaler 100. The electrical storage device 30 can be a battery, including but not limited to a lithium-ion, alkaline, zinc-carbon, nickel-metal hydride, or nickel-cadmium battery; a super capacitor; or a combination thereof. In the disposable embodiment, the electrical storage device 30 is not rechargeable, but, in the reusable embodiment, the electrical storage device 30 would be selected for its ability to recharge. In the disposable embodiment, the electrical storage device 30 is primarily selected to deliver a constant voltage over the life of the inhaler 100. Otherwise, the performance of the inhaler would degrade over time. Preferred electrical storage devices that are able to provide a consistent voltage output over the life of the device include lithium-ion and lithium polymer batteries.

The electrical storage device 30 has a first end 30a that generally corresponds to a positive terminal and a second end 30b that generally corresponds to a negative terminal. The negative terminal is extending to the first end 30a.

Because the electrical storage device 30 is located in the first portion 101 and the liquid reservoir structure 2 is located in the second portion 102, the joint needs to provide electrical communication between those components. In the present invention, electrical communication is established using at least an electrode or probe that is compressed together when the first portion 101 is tightened into the second portion 102.

In order for this embodiment to be reusable, the electrical storage device 30 is rechargeable. The casing 3 contains a charging port 32.

The integrated circuit 4 has a proximal end 4a and a distal end 4b. The positive terminal at the first end 30a of the electrical storage device 30 is in electrical communication with a positive lead of the flexible integrated circuit 4. The negative terminal at the second end 30b of the electrical storage device 30 is in electrical communication with a negative lead of the integrated circuit 4. The distal end 4b of the integrated circuit 4 comprise a microprocessor. The microprocessor is configured to process data from a sensor, to control a light, to direct current flow to means of ultrasonic vibrations 5 in the second portion 102, and to terminate current flow after a preprogrammed amount of time.

The sensor detects when the ultrasonic mist inhaler 100 is in use (when the user draws on the inhaler) and activates the microprocessor. The sensor can be selected to detect changes in pressure, air flow, or vibration. In a preferred embodiment, the sensor is a pressure sensor. In the digital embodiment, the sensor takes continuous readings which in turn requires the digital sensor to continuously draw current, but the amount is small and overall battery life would be negligibly affected.

Additionally, the integrated circuit 4 may comprise a H bridge, preferably formed by 4 MOSFETs to convert a direct current into an alternate current at high frequency.

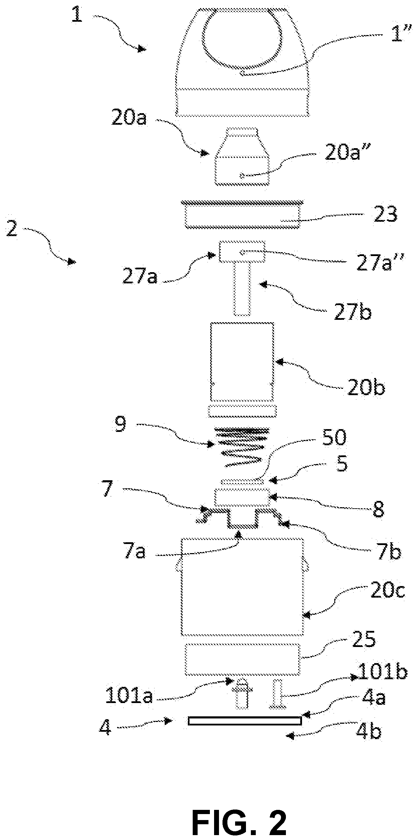

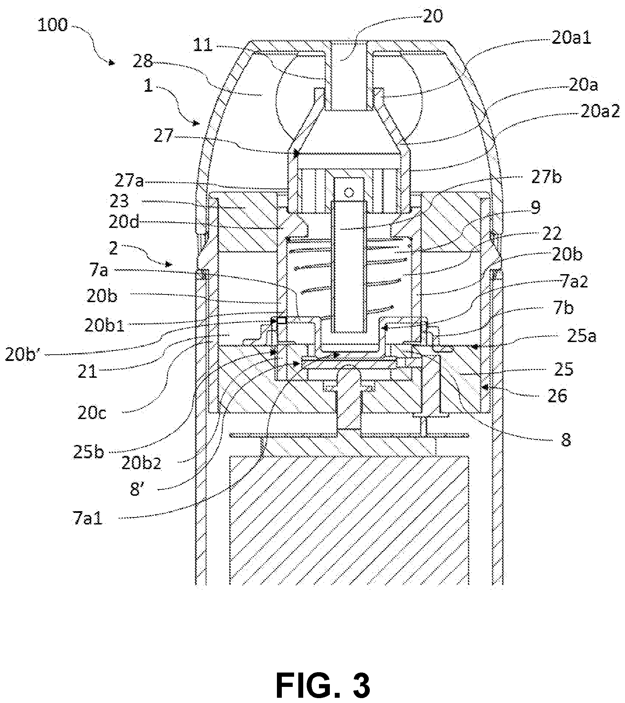

Referring to FIG. 2 and FIG. 3, illustrations of a liquid reservoir structure 2 according to an embodiment are shown. The liquid reservoir structure 2 comprises a liquid chamber 21 adapted to receive liquid to be atomized and a sonication chamber 22 in fluid communication with the liquid chamber 21.

In the embodiment shown, the liquid reservoir structure 2 comprises an inhalation channel 20 providing an air passage from the sonication chamber 22 toward the surroundings.

As an example of sensor position, the sensor may be located in the sonication chamber 22.

The inhalation channel 20 has a frustoconical element 20a and an inner container 20b.

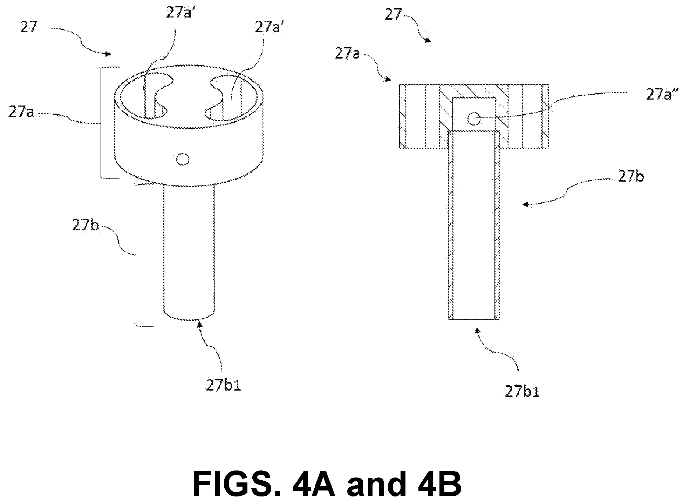

As depicted in Figure 4A and 4B, further the inhalation channel 20 has an airflow member 27 for providing air flow from the surroundings to the sonication chamber 22.

The airflow member 27 has an airflow bridge 27a and an airflow duct 27b made in one piece, the airflow bridge 27a having two airway openings 27a' forming a portion of the inhalation channel 20 and the airflow duct 27b extending in the sonication chamber 22 from the airflow bridge 27a for providing the air flow from the surroundings to the sonication chamber.

The airflow bridge 27a cooperates with the frustoconical element 20a at the second diameter 20a2.

The airflow bridge 27a has two opposite peripheral openings 27a" providing air flow to the airflow duct 27b.

The cooperation with the airflow bridge 27a and the frustoconical element 20a is arranged so that the two opposite peripheral openings 27a" cooperate with complementary openings 20a" in the frustoconical element 20a.

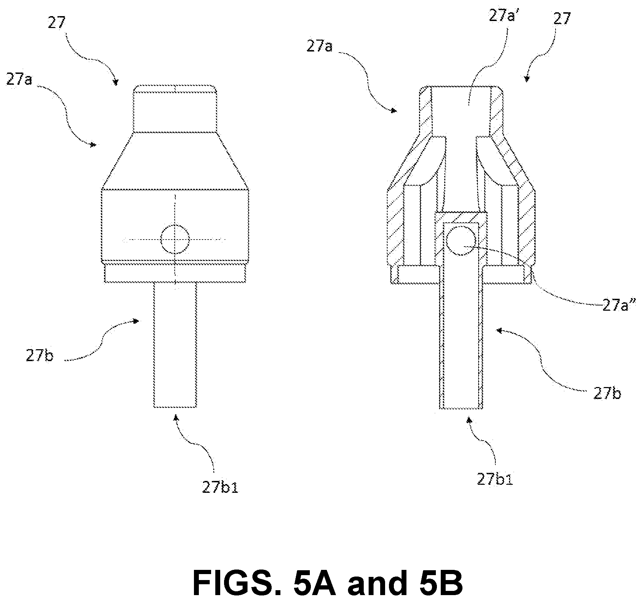

In the embodiment depicted in figures 5A and 5B, the airflow member 27 and the frustoconical element 20a are made in one piece.

The mouthpiece 1 and the frustoconical element 20a are radially spaced and an airflow chamber 28 is arranged between them.

As depicted in FIG. 1 and 2, the mouthpiece 1 has two opposite peripheral openings 1".

The peripheral openings 27a", 20a", 1" of the airflow bridge 27a, the frustoconical element 20a and the mouthpiece 1 directly supply maximum air flow to the sonication chamber 22.

The frustoconical element 20a includes an internal passage, aligned in the similar direction as the inhalation channel 20, having a first diameter 20a1 less than that of a second diameter 20a2, such that the internal passage reduces in diameter over the frustoconical element 20a.

The frustoconical element 20a is positioned in alignment with the means of ultrasonic vibrations 5 and a capillary element 7, wherein the first diameter 20a1 is linked to an inner duct 11 of the mouthpiece 1 and the second diameter 20a2 is linked to the inner container 20b.

The inner container 20b has an inner wall delimiting the sonication chamber 22 and the liquid chamber 21.

The liquid reservoir structure 2 has an outer container 20c delimiting the outer wall of the liquid chamber 21.

The inner container 20b and the outer container 20c are respectively the inner wall and the outer wall of the liquid chamber 21.

The liquid reservoir structure 2 is arranged between the mouthpiece 1 and the casing 3 and is detachable from the mouthpiece 1 and the casing 3.

The liquid reservoir structure 2 and the mouthpiece 1 or the casing 3 may include complimentary arrangements for engaging with one another; further such complimentary arrangements may include one of the following: a bayonet type arrangement; a threaded engaged type arrangement; a magnetic arrangement; or a friction fit arrangement; wherein the liquid reservoir structure 2 includes a portion of the arrangement and the mouthpiece 1 or the casing 3 includes the complimentary portion of the arrangement.

In the reusable embodiment, the components are substantially the same. The differences in the reusable embodiment vis-a-vis the disposable embodiment are the accommodations made to replace the liquid reservoir structure 2.

As shown in FIG. 3, the liquid chamber 21 has a top wall 23 and a bottom wall 25 closing the inner container 20b and the outer container 20c of the liquid chamber 21.

The capillary element 7 is arranged between a first section 20b1 and a second section 20b2 of the inner container 20b.

The capillary element 7 has a flat shape extending from the sonication chamber to the liquid chamber.

As depicted in FIG. 2 or 3, the capillary element 7 comprises a central portion 7a in U-shape and a peripheral portion 7b in L-shape.

The L-shape portion 7b extends into the liquid chamber 21 on the inner container 20b and along the bottom wall 25.

The U-shape portion 7a is contained into the sonication chamber 21. The U-shape portion 7a on the inner container 20b and along the bottom wall 25.

In the ultrasonic mist inhaler, the U-shape portion 7a has an inner portion 7a1 and an outer portion 7a2, the inner portion 7a1 being in surface contact with an atomization surface 50 of the means of ultrasonic vibrations 5 and the outer portion 7a2 being not in surface contact with the means of ultrasonic vibrations 5.

The bottom wall 25 of the liquid chamber 21 is a bottom plate 25 closing the liquid chamber 21 and the sonication chamber 22. The bottom plate 25 is sealed, thus preventing leakage of liquid from the sonication chamber 22 to the casing 3.

The bottom plate 25 has an upper surface 25a having a recess 25b on which is inserted an elastic member 8. The means of ultrasonic vibrations 5 are supported by the elastic member 8. The elastic member 8 is formed from an annular plate-shaped rubber having an inner hole 8' wherein a groove is designed for maintaining the means of ultrasonic vibrations 5.

The top wall 23 of the liquid chamber 21 is a cap 23 closing the liquid chamber 23.

The top wall 23 has a top surface 23 representing the maximum level of the liquid that the liquid chamber 21 may contain and the bottom surface 25 representing the minimum level of the liquid in the liquid chamber 21.

The top wall 23 is sealed, thus preventing leakage of liquid from the liquid chamber 21 to the mouthpiece 1.

The top wall 23 and the bottom wall 25 are fixed to the liquid reservoir structure 2 by means of fixation such as screws, glue or friction.

As depicted in FIG. 3, the elastic member 8 is in line contact with the means of ultrasonic vibrations 5 and prevents contact between the means of ultrasonic vibrations 5 and the inhaler walls, suppression of vibrations of the liquid reservoir structure are more effectively prevented. Thus, fine particles of the liquid atomized by the atomizing member can be sprayed farther.

As depicted in FIG. 3, the inner container 20b has openings 20b' between the first section 20b1 and the second section 20b2 from which the capillary element 7 is extending from the sonication chamber 21. The capillary element 7 absorbs liquid from the liquid chamber 21 through the apertures 20b'. The capillary element 7 is a wick. The capillary element 7 transports liquid to the sonication chamber 22 via capillary action. Preferably the capillary element 7 is made of bamboo fibers; however, cotton, paper, or other fiber strands could be used for a wick material.

In one embodiment of the ultrasonic mist inhaler 100, as can be seen in FIG. 3, the means of ultrasonic vibrations 5 are disposed directly below the capillary element 7.

The means of ultrasonic vibrations 5 may be a transducer. For example, the means of ultrasonic vibrations 5 may be a piezoelectric transducer, preferably designed in a circular plate-shape. The material of the piezoelectric transducer is preferably in ceramic.

A variety of transducer materials can also be used for the means of ultrasonic vibrations 5.

The end of the airflow duct 27b1 faces the means of ultrasonic vibrations 5. The means of ultrasonic vibrations 5 are in electrical communication with electrical contactors 101a, 101b. It is noted that, the distal end 4b of the integrated circuit 4 has an inner electrode and an outer electrode. The inner electrode contacts the first electrical contact 101a which is a spring contact probe, and the outer electrode contacts the second electrical contact 101b which is a side pin. Via the integrated circuit 4, the first electrical contact 101a is in electrical communication with the positive terminal of the electrical storage device 30 by way of the microprocessor, while the second electrical contact 101b is in electrical communication with the negative terminal of the electrical storage device 30.

The electrical contacts 101a, 101b crossed the bottom plate 25. The bottom plate 25 is designed to be received inside the perimeter wall 26 of the liquid reservoir structure 2. The bottom plate 25 rests on complementary ridges, thereby creating the liquid chamber 21 and sonication chamber 22.

The inner container 20b comprises a circular inner slot 20d on which a mechanical spring is applied.

By pushing the central portion 7a1 onto the means of ultrasonic vibrations 5, the mechanical spring 9 ensures a contact surface between them.

The liquid reservoir structure 2 and the bottom plate 25 can be made using a variety of thermoplastic materials.

When the user draws on the ultrasonic mist inhaler 100, an air flow is drawn from the peripheral openings 1" and penetrates the airflow chamber 28, passes the peripheral openings 27a" of the airflow bridge 27a and the frustoconical element 20a and flows down into the sonication chamber 22 via the airflow duct 27b directly onto the capillary element 7. At the same time, the liquid is drawn from the reservoir chamber 21 by capillarity, through the plurality of apertures 20b', and into the capillary element 7. The capillary element 7 brings the liquid into contact with the means of ultrasonic vibrations 5 of the inhaler 100. The user's draw also causes the
pressure sensor to activate the integrated circuit 4, which directs current to the means of ultrasonic vibrations 5. Thus, when the user draws on the mouthpiece 1 of the inhaler 100, two actions happen at the same time. Firstly, the sensor activates the integrated circuit 4, which triggers the means of ultrasonic vibrations 5 to begin vibrating. Secondly, the draw reduces the pressure outside the reservoir chamber 21
such that flow of the liquid through the apertures 20b' begins, which saturates the capillary element 7. The capillary element 7 transports the liquid to the means of ultrasonic vibrations 5, which causes bubbles to form in a capillary channel by the means of ultrasonic vibrations 5 and mist the liquid. Then, the mist liquid is drawn by the user.

The ultrasonic mist inhaler 100 of the present disclosures is a more powerful version of current portable medical nebulizers, in the shape and size of current e-cigarettes and with a particular structure for effective vaporization. It is a healthier alternative to cigarettes and current e-cigarettes products.

The ultrasonic mist inhaler 100 of the present disclosures has particular applicability for those who use electronic inhalers as a means to quit smoking and reduce their nicotine dependency. The ultrasonic mist inhaler 100 provides a way to gradually taper the dose of nicotine.

Other embodiments of the invented ultrasonic mist inhaler 100 are easily envisioned, including medicinal delivery devices.

It is to be understood that the above description is intended to be illustrative, and not restrictive. For example, the above-described embodiments may be used in combination with each other.

## Claims

1. An ultrasonic mist inhaler, comprising:
a liquid reservoir structure (2) comprising a liquid chamber (21) adapted to receive liquid to be atomized,
a sonication chamber (22) in fluid communication with the liquid chamber (21),
a means of ultrasonic vibrations (5) which is configured to generate ultrasonic waves within the sonication chamber (22);
a mouthpiece (1) for inhaling the mist;
an airflow member (27) which is provided within part of the liquid reservoir structure (2), wherein the airflow member (27) comprises:
an airflow bridge (27a) having at least one airway opening (27a') which forms part of an inhalation channel (20) from the sonication chamber (22) to a mouthpiece (1), and
an elongate airflow duct (27b) having a first end which is coupled to the airflow bridge (27a) and a second end which is positioned adjacent to and facing the means of ultrasonic vibrations (5),
wherein the airflow bridge (27a) further comprises a peripheral opening (27a") which provides an air flow path from the surroundings of the ultrasonic mist inhaler (100) to an airflow chamber (28), and then to the first end of the airflow duct (27b), such that the airflow member (27) provides air flow from the surroundings through the airflow duct (27b) to the sonication chamber (22) and out from the sonication chamber (22) through the inhalation channel (20) upon inhalation, wherein the airflow bridge (27a) is engaged with a frustoconical element (20a) which comprises at least a peripheral opening (20a") aligned with the peripheral opening (27a") of the airflow bridge (27a), and wherein the airflow chamber (28) is arranged between the mouthpiece (1) and the frustoconical element (20a).

2. The ultrasonic mist inhaler according to claim 1, wherein the mouthpiece (1) comprises at least a peripheral opening (1") aligned with the peripheral opening (27a") of the airflow bridge (27a) and the peripheral opening (20a") of the frustoconical element (20a).

3. The ultrasonic mist inhaler according to any of the preceding claims, wherein a capillary element (7) is arranged between the liquid chamber (21) and the sonication chamber (22).

4. The ultrasonic mist inhaler according to claim 3, wherein the second end of the airflow duct (27b) extends in the sonication chamber (22) and is close to or at the capillary element (7).

5. The ultrasonic mist inhaler according to any of the preceding claims, wherein the airflow bridge (27a) and the airflow duct (27b) are made in one piece.

6. The ultrasonic mist inhaler according to the any of the preceding claims, wherein said liquid to be received in the liquid chamber (21) comprises 57-70 % (w/w) vegetable glycerin and 30-43% (w/w) propylene glycol, said propylene glycol including nicotine and flavorings.

## Patentansprüche

1. Ultraschallnebelinhalator, umfassend:
eine Flüssigkeitsreservoirstruktur (2), umfassend eine Flüssigkeitskammer (21), die angepasst ist, um eine zu zerstäubende Flüssigkeit aufzunehmen,
eine Beschallungskammer (22) in Fluidverbindung mit der Flüssigkeitskammer (21),
ein Mittel für Ultraschallschwingungen (5), das konfiguriert ist, um Ultraschallwellen innerhalb der Beschallungskammer (22) zu erzeugen;
ein Mundstück (1) zum Inhalieren des Nebels;
ein Luftstromelement (27), das innerhalb eines Teils der Flüssigkeitsbehälterstruktur (2) bereitgestellt ist, wobei das Luftstromelement (27) umfasst:
eine Luftstrombrücke (27a), die mindestens eine Luftwegöffnung (27a') aufweist, die einen Teil eines Inhalationskanals (20) von der Beschallungskammer (22) zu einem Mundstück (1) ausbildet, und
einen länglichen Luftstromkanal (27b), der ein erstes Ende, das mit der Luftstrombrücke (27a) verbunden ist, und ein zweites Ende aufweist, das angrenzend an das Mittel für Ultraschallschwingungen (5) und gegenüber diesem angeordnet ist,
wobei die Luftstrombrücke (27a) ferner eine Randöffnung (27a") umfasst, die einen Luftstrompfad von der Umgebung des Ultraschallnebelinhalators (100) zu einer Luftstromkammer (28) und dann zu dem ersten Ende des Luftstromkanals (27b) derart bereitstellt, dass das Luftstromelement (27) bei Inhalieren einen Luftstrom von der Umgebung durch den Luftstromkanal (27b) zu der Beschallungskammer (22) und aus der Beschallungskammer (22) durch den Inhalationskanal (20) heraus bereitstellt, wobei die Luftstrombrücke (27a) mit einem kegelstumpfförmigen Element (20a) in Eingriff steht, das mindestens eine Randöffnung (20a") umfasst, die mit der Randöffnung (27a") der Luftstrombrücke (27a) ausgerichtet ist, und wobei die Luftstromkammer (28) zwischen dem Mundstück (1) und dem kegelstumpfförmigen Element (20a) angeordnet ist.

2. Ultraschallnebelinhalator nach Anspruch 1, wobei das Mundstück (1) mindestens eine periphere Öffnung (1") aufweisen, die zu der peripheren Öffnung (27a") der Luftstrombrücke (27a) und der peripheren Öffnung (20a") des kegelstumpfförmigen Elements (20a) ausgerichtet ist.

3. Ultraschallnebelinhalator nach einem der vorstehenden Ansprüche, wobei zwischen der Flüssigkeitskammer (21) und der Beschallungskammer (22) ein Kapillarelement (7) angeordnet ist.

4. Ultraschallnebelinhalator nach Anspruch 3, wobei das zweite Ende des Luftstromkanals (27b) sich in die Beschallungskammer (22) erstreckt und sich in der Nähe des Kapillarelements (7) oder an diesem befindet.

5. Ultraschallnebelinhalator nach einem der vorstehenden Ansprüche, wobei die Luftstrombrücke (27a) und der Luftstromkanal (27b) aus einem Stück gefertigt sind.

6. Ultraschallnebelinhalator nach einem der vorstehenden Ansprüche, wobei die in der Flüssigkeitskammer (21) aufzunehmende Flüssigkeit zu 57-70 Gew.-% pflanzliches Glycerin und zu 30-43 Gew.-% Propylenglycol umfasst, wobei das Propylenglycol Nicotin und Aromastoffe einschließt.

## Revendications

1. Inhalateur de brume ultrasonique, comprenant :
une structure de réservoir de liquide (2) comprenant une chambre à liquide (21) adaptée pour recevoir le liquide à atomiser,
une chambre de sonification (22) en communication fluidique avec la chambre à liquide (21),
un moyen de vibrations ultrasoniques (5) configuré pour générer des ondes ultrasoniques à l'intérieur de la chambre de sonification (22) ;
un embout buccal (1) pour l'inhalation de la brume ;
un élément de circulation d'air (27) qui est fourni à l'intérieur d'une partie de la structure de réservoir de liquide (2), dans lequel l'élément de circulation d'air (27) comprend :
un pont d'écoulement d'air (27a) ayant au moins une ouverture de passage d'air (27a') qui fait partie d'un canal d'inhalation (20) allant de la chambre de sonification (22) à un embout buccal (1), et
un conduit d'écoulement d'air allongé (27b) ayant une première extrémité accouplée au pont d'écoulement d'air (27a) et une seconde extrémité positionnée de manière adjacente et face au moyen de vibrations ultrasoniques (5),
dans lequel le pont d'écoulement d'air (27a) comprend en outre une ouverture périphérique (27a") qui fournit un chemin d'écoulement d'air depuis l'environnement de l'inhalateur de brume ultrasonique (100) vers une chambre d'écoulement d'air (28), puis vers la première extrémité du conduit d'écoulement d'air (27b), de sorte que l'élément d'écoulement d'air (27) fournit un écoulement d'air depuis l'environnement à travers le conduit d'écoulement d'air (27b) vers la chambre de sonification (22) et hors de la chambre de sonification (22) à travers le canal d'inhalation (20) au moment de l'inhalation, dans lequel le pont d'écoulement d'air (27a) est en prise avec un élément tronconique (20a) qui comprend au moins une ouverture périphérique (20a") alignée avec l'ouverture périphérique (27a") du pont d'écoulement d'air (27a), et dans lequel la chambre d'écoulement d'air (28) est disposée entre l'embout buccal (1) et l'élément tronconique (20a).

2. Inhalateur de brume ultrasonique selon la revendication 1, dans lequel l'embout buccal (1) comprend au moins une ouverture périphérique (1") alignée avec l'ouverture périphérique (27a") du pont d'écoulement d'air (27a) et l'ouverture périphérique (20a") de l'élément tronconique (20a).

3. Inhalateur de brume ultrasonique selon l'une quelconque des revendications précédentes, dans lequel un élément capillaire (7) est disposé entre la chambre à liquide (21) et la chambre de sonification (22).

4. Inhalateur de brume ultrasonique selon la revendication 3, dans lequel la seconde extrémité du conduit d'écoulement d'air (27b) s'étend dans la chambre de sonification (22) et est proche ou au niveau de l'élément capillaire (7).

5. Inhalateur de brume ultrasonique selon l'une quelconque des revendications précédentes, dans lequel le pont d'écoulement d'air (27a) et le conduit d'écoulement d'air (27b) sont fabriqués en une seule pièce.

6. Inhalateur de brume ultrasonique selon l'une quelconque des revendications précédentes, dans lequel ledit liquide à recevoir dans la chambre à liquide (21) comprend de 57 à 70 % (p/p) de glycérine végétale et de 30 à 43 % (p/p) de propylène glycol, ledit propylène glycol comportant de la nicotine et des arômes.
